# EUROPEAN PATENT APPLICATION

(11) **EP 4 424 307 A1**
(43) Date of publication of application: **04.09.2024**
(21) Application number: 23159875.6
(22) Date of filing: 03.03.2023
(51) Int. Cl.: A61K 31/198, A61P 1/16, A61P 13/12

(54) **PROTECTIVE AGENT FOR USE IN PERITONEAL DIALYSIS**

(71) Applicant: Zytoprotec GmbH, 1090 Wien (AT)
(72) Inventor: AUFRICHT, Christoph, 3400 Klosterneuburg-Weidling (AT); EIBENSTEINER, Fabian, 1090 Wien (AT); HERZOG, Rebecca, 1090 Wien (AT); KRATOCHWILL, Klaus, 1090 Wien (AT)
(74) Representative: Schwarz & Partner Patentanwälte GmbH

(57) **Abstract**

The present invention relates to a protective agent selected from the group consisting of L-glutamine, L-alanyl-L-glutamine, L-glutaminyl-L-alanine, L-glutaminyl-L-glycine, L-glycinyl-L-glutamine or mixtures thereof, for the specific use in the prevention and/or treatment, in the course of a peritoneal dialysis treatment, of
(A) liver diseases induced by said peritoneal dialysis treatment and/or
(B) diseases associated with end-stage kidney disease (ESKD) mediated by liver dysfunction induced by said peritoneal dialysis treatment,
whereby said protective agent is administered by intraperitoneal administration.

## Description

The present invention relates to a protective agent for use in peritoneal dialysis treatment as well as its specific therapeutic uses and methods of treatments employing said protective agent.

Chronic kidney disease (CKD) and loss of kidney function results in retention of uremic toxins and fluid overload, most pronounced in patients with end-stage kidney disease (ESKD, (1)). ESKD is also strongly associated with an increased risk for systemic inflammation, including metaflammation which is defined as a chronic low-grade inflammatory state induced by alterations in metabolism. Furthermore, ESKD is associated with atherosclerosis, and has been related to cardiovascular disease (CVD, (2)).

Peritoneal dialysis (PD) is a home-based kidney replacement therapy for ESKD with similar survival in comparison to hemodialysis (HD, (3)). With the use of a sterile procedure, peritoneal dialysis fluid (PDF) is instilled into the peritoneal cavity and removed after a few hours of dwell time. Driven mainly by hyperosmolar glucose-based or colloid-based PDF pressure gradients, PD reduces uremic toxins and fluid overload via small solute diffusion and ultrafiltration of water across the peritoneal membrane. Although providing life-saving kidney replacement, PD also introduces typical procedure related complications adding to uremic morbidities of ESKD, (3). Chronic exposure to PDF results in high intraperitoneal loads of glucose and its degradation products (GDPs), associated with inflammatory and vascular changes (4,5) and potentially promoting metabolic and cardiovascular risk profiles (6).

Most commercially available PDFs contain glucose monohydrate as their main osmolar agent. Alternatively PDFs may contain glucose polymers (i.e., icodextrin) or colloid-osmotic substances (e.g., amino-acids, peptides).

Several clinical and experimental observations have shown that PDF is cytotoxic, associated with a risk of technical failure of up to 30% with long term PD treatment (7).

WO 2008/106702A1 provides a glucose-based peritoneal fluid containing a protective agent in the form of L-glutamine and dipeptides which are capable of releasing L-glutamine, said dipeptide being L-glutaminyl-L-glycine, L-glycinyl-L-glutamine, L-glutaminyl-L-alanine or L-alanyl-L-glutamine, or a mixture of two or more of said dipeptides, wherein the concentration of said dipeptide in the dialysis fluid is 2 mM to 25 mM.

According to this patent application, it was found that these dipeptides, especially L-alanyl-L-glutamine (in the following abbreviated both as Alanyl-Glutamine or also as "Ala/Gln"), contribute to the prevention of Technical Failure.

The common concept of these protective agents is their capability to release L-glutamine. Thus, while in the following mainly reference to Alanyl-Glutamine is made, it is to be expected that the reported effects also will achieved when using other protective agents of the afore-mentioned group.

Several publications have further investigated the effect of Ala/Gln in glucose-based PDFs (8-26).

The liver is increasingly recognized as central hub linking metabolism and CVD (27). In order for the liver to tolerate exposure to gut derived microbial and dietary molecules, metabolic activities and associated inflammatory processes are tightly controlled. Stimulation occurs when clearance of hepatotropic pathogens or toxic products of metabolic activity is required (28). Several studies have shown epidemiological correlations between CKD and chronic liver disease, with recent emphasis on metabolic stress contributing to the kidney-liver crosstalk (29,30).

In CKD, the liver might represent both victim, developing specific pathologies such as metabolic (dysfunction)-associated fatty liver disease (MAFLD), and aggressor, mediating a milieu driving CKD associated CVD. The liver's central role in detection and response to extra- and intrahepatic signals is accomplished by the acute phase response resulting in amplification of inflammatory stimuli by orders of magnitude. Hepatocytes produce acute phase proteins (e.g., complement proteins, C-reactive protein [CRP]) which then promote systemic inflammation via direct effector functions (27,28).

Although these pathomechanisms are highly prevalent in ESKD patients treated with PD, the inventors of the present invention are not aware of mechanistic studies that link PD with liver diseases or elucidate the role of PD induced dysfunction of the liver in conditions associated with PD, such as CVD.

Clinical reports linking PD and the liver are sparse and inconclusive. Historic reports demonstrated subcapsular steatosis hepatis in PD patients and linked its occurrence to the concurrent use of intraperitoneal insulin with glucose-base PDFs, associated with higher peritoneal transfer rates and higher body weight (31,32). A small cross-sectional study reported high prevalence of chronic liver disease in PD patients, associated with high risk for atherosclerosis and therefore CVD (33). Liver enzymes have been reported to be higher in PD patients in comparison to HD patients in some studies (34), but could not be found in others (35). Liberato et al. discuss that their observed increase of Gamma-Glutamyl-Transferase (GGT) above upper normative levels in both dialysis groups could be related to the malnutrition-inflammation-atherosclerosis syndrome (34), thereby in close relation to CVD. Therefore, it was unclear in clinical PD whether there is a specific effect of PD on the liver, as well as the role of PD induced dysfunction of the liver in inflammatory responses.

It is an object of the present invention to provide an amelioration of the side effects associated with PD.

This object is solved by the subject matter of claim 1.

Further preferred embodiments of the invention are disclosed in the dependent claims.

### SHORT DESCRIPTION OF THE DRAWINGS

**Fig. 1** | Peritoneal dialysis induced hepatic inflammatory response. Control and uremic mice with and without PD.
   Inflammation score and non-alcoholic fatty liver disease (NAFLD) activity score (NAS). P-values calculated by Mann Whitney U test, no multiplicity correction.
   a, Changes in healthy animals
   b, Changes in the uremic animal model
**Fig. 2** | Effect of Alanyl-Glutamine supplementation to PDF on PD induced hepatic inflammatory response. Control mice, uremic mice with PD, and uremic mice with PD and AlaGln supplementation. P-values calculated by Mann Whitney U test, no multiplicity correction:
   a, Inflammation score and NAFLD activity score (NAS);
   b, Percentage area of Oil Red O stained tissue (= steatosis) on liver sections
   c, Percentage area of Sirius Red stained tissue (= fibrosis) on liver sections.
**Fig. 3** | Effect of administration of Alanyl-Glutamine on specific biomarkers related to cardiovascular disease. Control mice, uremic mice with PD, and uremic mice with PD and AlaGln supplementation.
   Abundance of APOH, CFB, CRP in liver tissue assessed by mass spectrometry proteomics analysis.
**Fig. 4** | Association of changes in different liver parameters with one-another as well as with changes in parameters of systemic inflammation due to Alanyl-Glutamine treatment in patients on chronic PD. Secondary analysis of a prospective, multi-center phase II trial of the effect of Alanyl-Glutamine supplementation to PDF in patients on chronic PD regarding clinical parameters of liver injury.
   a, Spearman rank correlation, tiles colored black display positive correlation with a p-value<0.1 (no multiplicity correction). White tiles display non-significant (p-value≥0.1) positive correlation.
   b, Spearman rank correlation between changes of plasma Interleukin 6 and changes of GGT levels (rho=0.35, p=0.03, no multiplicity correction).

### DETAILLED DESCRIPTION OF THE INVENTION

The present inventors for the first time have analyzed the effect of PD itself on the liver of both uremic and non-uremic animals, and it was surprisingly found that PD, in particular with a glucose based PDF, itself induces liver diseases and, thus, also enhances the probability of diseases mediated by liver dysfunction.

In the inventors' study, uremic and non-uremic animals receiving PD allowed mechanistic studies to elucidate specific effects of PD on molecular responses of the liver in a closely controlled experimental model of CKD. The experimental data shows that PD treatment is associated with hepatic inflammation and increased acute phase response as novel treatment-specific adverse effect that might diminish its beneficial effects of reducing uremic toxemia and hyperhydration in ESKD patients. Furthermore, the experimental setting of PD in non-uremic animals in the inventor's study allows unique insights into specific PD procedure related pathomechanisms that can otherwise not be obtained. Interestingly, results in these experiments clearly corroborate differential expression of liver proteins related to hepatic acute phase response signaling without CKD as confounder.

These data indeed suggest a direct causal role of specific PD factors to induce hepatic inflammation and acute phase response signaling, and subsequent systemic inflammation, especially metaflammation, and CVD in patients on PD. PDF induced excess of energy and nutrients directly transferred to the liver (via the portalvenous runoff of the peritoneum) may result in metabolic stress, triggering metaflammation and alteration of immune responses thereby promoting disease, both liver disease and CVD.

In conclusion, the inventors have found direct evidence of PD-induced hepatic pathology and dysfunction. Applying an established chronic PD exposure model in both uremic and non-uremic mice, their histologic and proteomic findings directly link chronic intraperitoneal PDF exposure to hepatic inflammation and liver-mediated systemic inflammation, especially metaflammation, via the portal peritoneal-liver axis, regardless of CKD.

The inventors found that the protective agents as used per claim 1, especially Alanyl-Glutamine, upon intraperitoneal administration in the course of a peritoneal dialysis treatment, ameliorate the negative impact of the PDF on the liver.

The inventors investigated the potential hepatic effects of Alanyl-Glutamine supplementation in a chronic murine PD model. This treatment resulted in significant attenuation of PD-induced hepatic pathology and liver-mediated metaflammation. Histologically, evidence of Alanyl-Glutamine mediated reduction of PD-induced morphologic liver pathology was demonstrated by improved NAFLD activity scores (NAS) mainly driven by reduction of inflammatory infiltration. This morphologic improvement was associated with respective molecular responses, demonstrated in the murine liver proteome.

Alanyl-Glutamine has been shown to reduce hepatic reperfusion-injury in a rat model, to protect mice against LPS-induced acute liver injury, and to significantly attenuate steatohepatitis and fibrosis in a murine NAFLD model (36-38).

However, the use of Alanyl-Glutamine to directly influence PD-induced liver diseases and/or further liver-related negative consequences of PD has not been suggested so far.

Thus, in a first aspect the present invention provides a protective agent selected from the group consisting of L-glutamine, L-alanyl-L-glutamine, L-glutaminyl-L-alanine, L-glutaminyl-L-glycine, L-glycinyl-L-glutamine or mixtures thereof, for the specific use in the prevention and/or treatment, in the course of a peritoneal dialysis treatment, of
(A) liver diseases induced by said peritoneal dialysis treatment and/or
(B) diseases associated with end-stage kidney disease (ESKD) mediated by liver dysfunction induced by said peritoneal dialysis treatment,
whereby said protective agent is administered by intraperitoneal administration.

A further aspect of the present invention relates to the use of said protective agent in the prevention and/or treatment, in the course of a peritoneal dialysis treatment of said diseases (A) and/or (B).

A further aspect of the present invention relates to the use of said protective agent in the manufacture of a medicament for the treatment or prevention, in the course of a peritoneal dialysis treatment of said diseases (A) and/or (B).

Yet a further aspect of the present invention relates to a method of prevention and/or treatment of said diseases (A) and/or (B), by administering, in the course of a peritoneal dialysis treatment, said protective agent to a subject in need therefor via intraperitoneal administration.

The following discussion relates to any of the above aspects, *mutatis mutandis.*

In a preferred embodiment of the present invention, the liver diseases induced by peritoneal dialysis treatment (A) are chronic liver diseases, especially non-infectious liver inflammation.

Non-infectious liver inflammation includes diseases such as non-alcoholic fatty liver disease (NAFLD), non-alcoholic steato hepatitis (NASH) and metabolic (dyfunction)- associated fatty liver disease, (MAFLD).

In ESKD patients the pathophysiologic mechanisms linking PD and the liver cannot be disentangled from effects of kidney-liver crosstalk in CKD, and its chronic low-grade inflammatory factors and metabolic factors (associated with e.g. atherogenic dyslipidemia and dysglycemia, (30)). CKD shares many risk factors with fatty liver disease (e.g., obesity, hypertension, insulin resistance, type 2 diabetes mellitus, atherogenic dyslipidemia), wherefore the prevalence of CKD in the non-alcoholic fatty liver disease (NAFLD) population is significantly larger at approximately 20-55% in comparison to the non-NAFLD population with 5-35% (39). Furthermore, several studies have shown correlations between CKD and NAFLD, describing the broader phenotype of metabolic (dysfunction) associated fatty liver disease (MAFLD) with more emphasis on the pathogenic role of metabolic dysfunction, as well as increased risk for CVD by CKD, MAFLD, and their shared and non-shared risk factors (29,30,40-43). In a population-based study the positive association between the fatty liver index, a marker for hepatic steatosis, and CKD incidence was fully mediated by joint effects of the most important cardiometabolic risk factors (44). Markers of liver pathology were significantly correlated to serum CRP, ALT, AST, and lipid markers (i.e. Cholesterol, LDL, triglycerides), as well as to CVD in dialysis patients (45). Furthermore, parameters of liver function were positively correlated with poor outcomes, CVD, and all-cause mortality in Patients with ESKD and PD (46).

So far, no data of Alanyl-Glutamine effects on hepatic injury and liver-mediated systemic metaflammation (potentially driving CVD) has been reported in patients on PD.

Thus, in addition to above-mentioned experiments in an animal model, the inventors set out to conduct a secondary outcomes analysis of a recent clinical PD trial (in human patients, 8) to validate their experimental findings.

As a result of this novel analysis, the inventors were able to identify bulk reduction of clinical parameters of liver injury upon addition of Alanyl-Glutamine to PDF that reached statistical significance with LDH and the liver-specific ALT.

In the afore-mentioned trial (8), addition of Alanyl-Glutamine to PDF was also reported to attenuate systemic inflammation, as reflected by a tendency to reduce biomarkers such as hs-CRP and IL-6 , that reached statistical significance with IL-8 (8, Table 3).

The inventor's novel secondary analysis now additionally revealed a significant association between changes in markers of systemic inflammation (Interleukin 6) to changes of liver enzymes (GGT) as well as clinical markers of cell lysis (AST, LDH).

These findings also corroborate with the inventor's experimental findings in the murine model of treatment effects of Alanyl-Glutamine on regulated cell death (e.g., necroptosis signaling) and systemic inflammation, especially metaflammation, suggesting that Alanyl-Glutamine acts on the liver as both victim (sentinel) and aggressor (mediator) in patients on PD.

Taken together, the experimental findings and the novel secondary data analysis of a human clinical trial show that intraperitoneal treatment with Alanyl-Glutamine not only attenuates PD-induced hepatic inflammatory injury and liver-mediated systemic inflammation, especially metaflammation, in experimental systems, but also results in reduction of clinically relevant parameters of liver injury and systemic inflammation in patients on chronic PD.

In yet a further embodiment of the present invention said ESKD-associated diseases mediated by liver dysfunction (B) are selected from the group consisting of cardiovascular diseases, especially artherosclerotic cardiovascular diseases, including inflammation driven cardiovascular diseases and metaflammation driven cardiovascular diseases.

As set out above, the liver is increasingly recognized as central hub linking metabolism and CVD.

As discussed above, intraperitoneal treatment with Alanyl-Glutamine not only attenuates PD-induced hepatic inflammatory injury and liver-mediated systemic inflammation, especially metaflammation, in experimental systems, but also results in reduction of clinically relevant parameters of liver injury and systemic inflammation in patients on chronic PD.

The attenuation of the novel side effects of PD discovered by the inventors by addition of Alanyl-Glutamine to PDF provides a novel targeted therapeutic option in patients on PD to improve systemic inflammation, especially metaflammation, and thereby CVD outcomes.

This novel therapeutic option may provide a suitable alternative to the administration of anti-inflammatory biologicals, such as IL-6-specific antibodies.

In one embodiment of the present invention, the administration of the protective agent starts from the outset of said peritoneal dialysis treatment.

In another embodiment of the present invention administration of the protective agent starts after a period from the outset of said peritoneal dialysis treatment.

In this embodiment, the administration may start once indicators for liver diseases or liver-mediated systemic inflammation, especially metaflammation, associated with peritoneal dialysis treatment, are detected. Such indicators may be biomarkers, such as laboratory markers, and/or imaging findings.

As indicators for liver disease, serum markers of hepatobiliary injury (e.g., AST, ALT, GGT, ALP, Bilirubin) and/or imaging findings indicative of liver injury and/or hepatitis and/or fatty degeneration/deposition and/or fibrosis (e.g., MRI, CT, PET, hepatic elastography, Ultrasound) may be observed.

As indicators for inflammation/metaflammation, again serum markers (e.g., CRP, hs-CRP, IL-6, IL-8, Albumin, Fibrinogen, SAA) and/or a erythrocyte sedimentation rate and/or imaging findings (e.g., PET) may be observed.

As indicators for cardiovascular disease, especially inflammation/metaflammation driven cardiovascular disease, again biomarkers (e.g., CRP, hs-CRP, IL-6, IL-8, Fibrinogen, SAA) and/or imaging findings (e.g., Angiography, MRI, CT, PET, Ultrasound) may be observed.

According to the present invention, the protective agent is administered intraperitoneally to reach those parts of the human body which are directly affected by the PD treatment.

Interperitoneal administration may take place via intraperitoneal injection or infusion of the protective agent within a suitable pharmaceutical carrier as known to the skilled artisan.

The administration may be performed in an intermittent or continuous way.

In an especially preferred embodiment, the protective agent is administered as a component of a peritoneal dialysis fluid (PDF).

The PDF which contains the protective agent may preferably be the very same PDF which is administered to the patient for the PD treatment.

In this case, the body, already undergoing a PD treatment, is not exposed to yet a further different treatment solution.

The PDF containing the protective agent, in this embodiment may be administered (as the only PDF) from the outset of said PD treatment.

Alternatively, if treatment begins with another peritoneal dialysis fluid, said other PDF may be completely replaced by the fluid containing the protective agent, especially once indicators for liver diseases or liver-mediated systemic inflammation, especially metaflammation, are deteceted.

Preferably the fluid containing the protective agent is administered as the only PDF in the course of the PD treatment. This means that the patient is only treated with the PDF containing the protective agent.

In another embodiment, the fluid may be administered in combination with another peritoneal dialysis fluid. Said other peritoneal dialysis fluid may be selected from any available PDF that is compatible with the PDF containing the protective agent.

In yet a further embodiment of the present invention, said PDF containing the protective agent is based on glucose as the osmotic agent.

Preferably, the protective agent used according to the present invention or in the method of the present invention is L-alanyl-L-glutamine, optionally in mixture with one or more of the other protective agents.

### EXAMPLES

### Examples 1 and 2 - Murine test model

### METHODS

The inventors conducted a murine PD exposure experiment resembling chronic PD in healthy and uremic mice:
The experiment consisted of n=34 animals in six distinct groups.

The animals were split in the following groups:
1) Healthy control animals ("control")
2) Healthy animals receiving PD treatment (via an indwelling catheter) ("PD")
3) Healthy animals receiving PD treatment (via an indwelling catheter) supplemented with Alanyl-Glutamine ("PD+AG")
4) Uremic animals ("uremic")
5) Uremic animals receiving PD treatment (via an indwelling catheter) ("uremic PD")
6) Uremic animals receiving PD treatment (via an indwelling catheter) supplemented with Alanyl-Glutamine ("uremic PD+AG")

All animals of the uremic groups (Groups 4, 5, and 6) were subjected to left-sided 2/3 nephrectomy at day 0 of the study, and to complete right-sided nephrectomy at day 7 of the study, thereby inducing chronic kidney disease.

All animals receiving PD treatment (Groups 2, 3, 5, and 6) had their indwelling catheter implanted at day 7 of the study.

Animals in groups 2,3, 5, and 6 received daily PDF (Dianeal PD4 3.86% [Baxter, Deerfield, IL, USA]) injections via their indwelling catheter starting from day 14 over the course of six weeks, thereby inducing chronic PDF exposure.

For animals of groups 3 and 6 the daily PDF injections were supplemented with Alanyl-Glutamine (8 mM) over the course of six weeks.

At sacrifice (day 56 of the study), animal livers were subjected to histologic analysis.

NAFLD activity score (NAS) assessment was conducted as previously described (47) on hematoxylin & eosin (H&E) stained hepatic tissue on one facial field image per mouse.

Steatosis hepatis was assessed by semi-quantitative measurement of Oil Red O stained hepatic tissue with one facial field image per mouse.

Fibrosis hepatis was assessed by semi-quantitative measurement of Sirius Red stained hepatic tissue with one facial field image per mouse.

### EXAMPLE 1 - PD induces liver injury regardless of uremic status - Alanyl-Glutamine attenuates this effect of PD

Liver histology in H&E staining at the end of the experiment showed markedly increased numbers of inflammatory immune cell infiltrates, especially near interlobular veins of the portal venous system (the peritoneal runoff), together with increased areas of hepatocyte ballooning in PD treated animals.

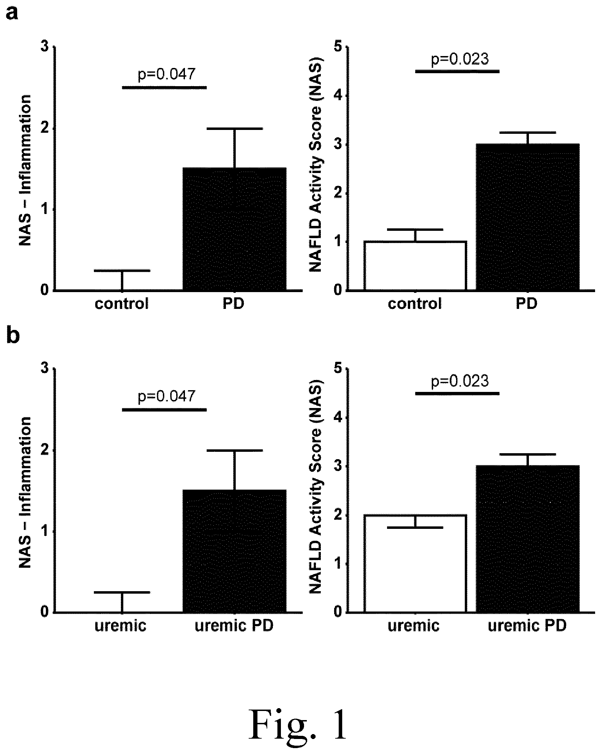

These findings are represented by significantly increased NAFLD activity scores (NAS) for mice that were treated with PD, as displayed in Fig. 1.

Fig. 1 a displays these changes in healthy animals whereas Fig. 1 b displays these changes in the uremic animal model. In both panels, mice with PD displayed significantly increased (p<0.05) NAFLD activity scores (NAS) in comparison to mice without PD.

Thus, this effect of PD was observed both for healthy and uremic animals, i.e. regardless of uremic status. These NAS increases were mainly caused by higher numbers of inflammatory infiltrates.

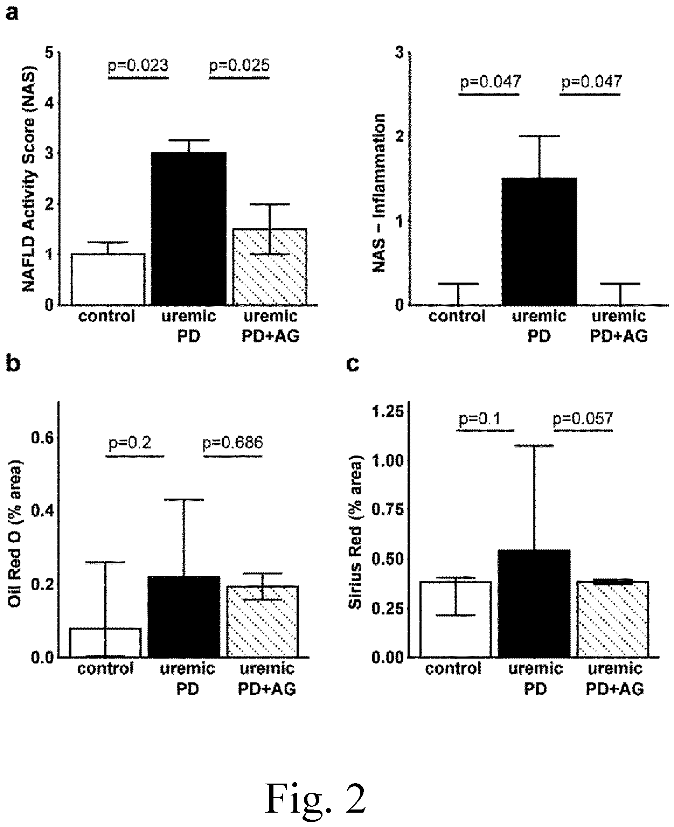

On the other hand, addition of Alanyl-Glutamine to the PDF resulted in markedly decreased numbers of inflammatory immune cell infiltrates and decreased areas of hepatocyte ballooning in H&E liver sections, semi-quantitatively expressed as significantly (p<0.05) decreased NAS in uremic mice. Again, this is mainly driven by the occurrence of inflammatory infiltrates (Fig. 2a). Furthermore, trends of improvement of PD induced steatosis (as indicated by % area of Oil Red O stained tissue, Fig. 2b) and fibrosis (as indicated by % area of Sirius Red stained tissue, Fig. 2c) hepatis can be observed by addition of Alanyl-Glutamine to the PDF.

### EXAMPLE 2 - PD treatment induces hepatic inflammatory response and acute phase response; again, Alanyl-Glutamine attenuates this effect

To search for effects of PD induced or associated hepatic molecular inflammatory responses as well as to discover potential novel treatment interventions, all animal livers of the murine experiment in Example 1 were subjected to mass spectrometry proteomics analysis.

The inventors extracted all identified proteins that were either part of the acute phase response signaling pathway or part of pathways associated with hepatic inflammation pathways using Ingenuity Pathway Analysis (EMEA Qiagen, Aarhus, Denmark). Of the subset of proteins that were identified by PLS-DA (partial least squares-discriminant analysis) to be most important for the discrimination of livers between animals with PD and without PD, 57 were significantly (p<0.05, linear model with Empirical Bayes shrinkage for variance stabilization) dysregulated by PD in comparison to control animals.

Supplementation of PDF with Alanyl-Glutamine was able to significantly alter (p<0.05, linear model with Empirical Bayes shrinkage for variance stabilization) the abundance of 11 of these acute-phase-response- and inflammation-associated proteins within the liver of uremic animals receiving PD treatment. These 11 proteins are part of acute-phase-response signaling (e.g., CFB, APOH, and RELA), glucocorticoid receptor signaling (e.g. HSPA5, NCOR1, NDUFA4, NDUFB3, SDHB, RELA, and IRF3), and necroptosis signaling (e.g., TIMM13, PPID, and IRF3) pathways.

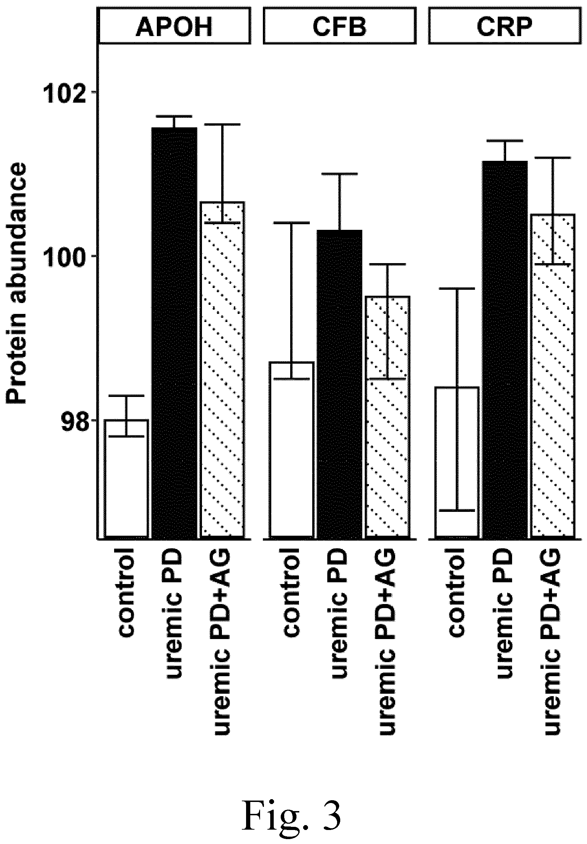

Fig. 3 shows this effect in graphical form on three selected biomarkers (APOH, CFB, and CRP) known for having a clinically relevant association with cardiovascular risk.

Thus, the inventors herein demonstrate a clear delineating effect of PD on hepatic inflammation and liver-mediated systemic inflammation, especially metaflammation, as well as amelioration thereof by Alanyl-Glutamine supplementation to PDF on hepatic protein level. The effect of Alanyl-Glutamine supplementation to PDF on PD-induced hepatic inflammation and liver-mediated systemic inflammation, especially metaflammation, was mainly achieved via regulation of proteins of the acute phase response signaling, glucocorticoid receptor signaling (regulation of NFκB and respective downstream effects), and necroptosis signaling pathways.

### EXAMPLE 3 - Alanyl-Glutamine supplementation to PDF improves hepatic (dys)function in human PD Patients

To further investigate the effect of Alanyl-Glutamine supplementation to PDF in human PD-induced hepatic (dys)function, the inventors conducted a secondary analysis of a prospective, multicenter, double-blinded, controlled, randomized, dual-period, 2-treatment, crossover, phase II, proof-of-concept study including eight Austrian centers for Alanyl-Glutamine supplementation to the PDF in patients on chronic PD.

For this purpose, the inventors included the liver tests (GOT/AST, GPT/ALT, GGT, LDH) that were obtained as safety data in all patients of the full analysis set, as the per-protocol analysis excluded patients with interfering relevance to local peritoneal effects only, which resulted in n=41 patients with chronic PD randomized to either the placebo first or Alanyl-Glutamine first arm of this cross-over study, for secondary analytical purposes.

This study cohort and its baseline characteristics have already been previously described by Vychytil et al (8).

Alanyl-Glutamine supplementation to PDF was able to significantly reduce clinical markers of liver injury. These effects of Alanyl-Glutamine in a human trial in patients on chronic PD are in good agreement with the effects of Alanyl-Glutamine on regulated cell death (e.g., necroptosis signaling) on protein-level in the murine experiments as described in Example 2. Effects of Alanyl-Glutamine supplementation to PDF on markers of liver (dys)function are displayed below in Table 1.

| | **Placebo** | **Alanyl-Glutamine** | **Difference Alanyl-Glutamine-placebo** | |
|---|---|---|---|---|
| **Parameter (unit)** | **Least squares mean (95% CI)** | **Least squares mean (95% CI)** | **estimate ± SE** | **p value** |
| *GOT*/*AST (U*/*L)*^{∗} | 1.26 (1.22 to 1.31) | 1.23 (1.19 to 1.27) | -0.04 ± 0.03 | 0.1877 |
| ***GPT*/*ALT (U*/*L)**** | **1.26 (1.20 to 1.31)** | **1.20 (1.15 to 1.25)** | **-0.06 ± 0.03** | **0.0466** |
| *GGT (U*/*L)*^{∗} | 1.43 (1.38 to 1.47) | 1.41 (1.37 to 1.46) | -0.02 ± 0.02 | 0.4901 |
| *LDH* ***(U*/*L)**** | **2.33 (2.30 to 2.35)** | **2.30 (2.27 to 2.32)** | **-0.03 ± 0.01** | **0.0206** |

| | | | | |
|---|---|---|---|---|
| ^{∗} *log10 transformed due to skewness* | | | | |

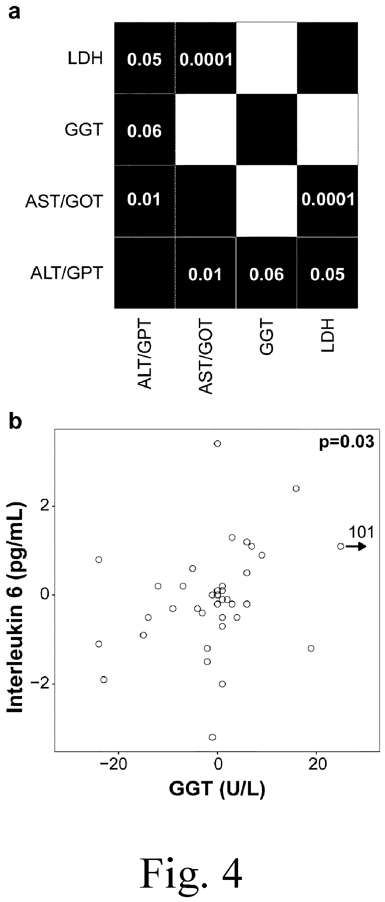

Furthermore, a positive correlation of the changes induced by Alanyl-Glutamine supplementation to PDF on different clinical diagnostic markers for hepatobiliary injury with one-another was observed. This is shown in Fig. 4 Panel a. As each marker coins different patterns of cellular injury within the hepatobiliary system, different levels of signficance for each correlation pair can be observed.

Panel b displays the positive significant correlation of the changes induced by Alanyl-Glutamine supplementation to PDF on one of the clinical diagnostic markers for hepatobiliary injury (GGT) and a clinical diagnostic marker for inflammation (IL-6). As mentioned above, systemic administration of anti-inflammatory biologicals, such as IL-6-specific antibodies, is currently developed as therapeutic option to treat metaflammation and reduce the risk of CVD. Thus, Fig. 4 links the observed reduction of clinical diagnostic markers for hepatobiliary injury by Alanyl-Glutamine supplementation to PDF (Table 1, Fig. 4 panel a) to the observed reduction of clinical diagnostic markers for inflammation, especially/including/and metaflammation (Fig. 4 panel b). This serves as validation for the observed mechanistic effects of Alanyl-Glutamine of the murine PD model of Examples 1 and 2 in a human clinical randomized placebo-controlled phase II trial.

Literature:
(1). Chronic kidney disease. Nat Rev Dis Primers 2017; 3: 17088.
(2). Excess mortality due to interaction between protein-energy wasting, inflammation and cardiovascular disease in chronic dialysis patients. Nephrol Dial Transplant 2008; 23(9): 2957-64.
(3). Peritoneal Dialysis. The New England journal of medicine 2021; 385(19): 1786-95.
(4). Complement Activation in Peritoneal Dialysis-Induced Arteriolopathy. J Am Soc Nephrol 2018; 29(1): 268-82.
(5). Glucose Derivative Induced Vasculopathy in Children on Chronic Peritoneal Dialysis. Circ Res 2021; 129(5): e102-e18.
(6). ISPD Cardiovascular and Metabolic Guidelines in Adult Peritoneal Dialysis Patients Part I - Assessment and Management of Various Cardiovascular Risk Factors. Perit Dial Int 2015; 35(4): 379-87.
(7). Safety and biocompatibility in perfect balance. Fresenius Medical Care Deutschland GmbH. 2006.
(8). A randomized controlled trial of alanyl-glutamine supplementation in peritoneal dialysis fluid to assess impact on biomarkers of peritoneal health. Kidney Int. 2018 Dec;94(6):1227-1237.
(9). Biomarker research to improve clinical outcomes of peritoneal dialysis: consensus of the European Training and Research in Peritoneal Dialysis (EuTRiPD) network. Kidney Int. 2017.
(10). Functional and Transcriptomic Characterization of Peritoneal Immune-Modulation by Addition of Alanyl-Glutamine to Dialysis Fluid. Sci Rep. 2017 Jul 24;7(1):6229
(11). Addition of Alanyl-Glutamine to Dialysis Fluid Restores Peritoneal Cellular Stress Responses - A First-In-Man Trial. PloS One. 2016 Oct 21;11(10):e0165045.
(12). Dynamic O-linked N-acetylglucosamine modification of proteins affects stress responses and survival of mesothelial cells exposed to peritoneal dialysis fluids. J Am Soc Nephrol. 2014 Dec;25(12):2778-88.
(13). Alanyl-glutamine dipeptide restores the cytoprotective stress proteome of mesothelial cells exposed to peritoneal dialysis fluids. Nephrol Dial Transplant. 2012 Mar;27(3):937-46.
(14). Peritoneal dialysate fluid composition determines heat shock protein expression patterns in human mesothelial cells. Kidney Int. 2001 Nov;60(5):1930-7.
(15). Alanyl-Glutamine Restores Tight Junction Organization after Disruption by a Conventional Peritoneal Dialysis. Biomolecules. 2020 Aug 13;10(8).
(16). The Peritoneal Surface Proteome in a Model of Chronic Peritoneal Dialysis Reveals Mechanisms of Membrane Damage and Preservation. Front Physiol. 2019 May 14;10:472.
(17). Does alanyl-glutamine supplementation offer potential to improve peritoneal dialysate biocompatibility? Kidney Int. 2018 Dec;94(6):1050-1052.
(18). Injury-induced inflammation and inadequate HSP expression in mesothelial cells upon repeat exposure to dual-chamber bag peritoneal dialysis fluids. Int J Artif Organs. 2015 Oct;38(10):530-6.
(19). Is there such a thing as biocompatible peritoneal dialysis fluid? Pediatr Nephrol. 2016 Oct 8.
(20). HSP-mediated cytoprotection of mesothelial cells in experimental acute peritoneal dialysis. Perit Dial Int. 2010 May-Jun;30(3):294-9.
(21). Peritoneal dialysis fluids can alter HSP expression in human peritoneal mesothelial cells. Nephrol Dial Transplant. 2011 Mar;26(3):1046-52.
(22). Overexpression of HSP-72 confers cytoprotection in experimental peritoneal dialysis. Kidney Int. 2004Dec;66(6):2300-7.
(23). The dipeptide alanyl-glutamine ameliorates peritoneal fibrosis and attenuates IL-17 dependent pathways during peritoneal dialysis. Kidney Int. 2016 Mar;89(3):625-35.
(24). Effects of Alanyl-Glutamine Treatment on the Peritoneal Dialysis Effluent Proteome Reveal Pathomechanism-Associated Molecular Signatures. Mol Cell Proteomics. 2018 Mar;17(3):516-532.
(25). Peritoneal Dialysis Fluid Supplementation with Alanyl-Glutamine Attenuates Conventional Dialysis Fluid-Mediated Endothelial Cell Injury by Restoring Perturbed Cytoprotective Responses. Biomolecules. 2020 Dec 15;10(12).
(26). Targeted Metabolomic Profiling of Peritoneal Dialysis Effluents Shows Antioxidative Capacity of Alanyl-Glutamine. Front Physiol. 2019 Jan 21;9:1961.
(27). Metabolic Inflammation-A Role for Hepatic Inflammatory Pathways as Drivers of Comorbidities in Nonalcoholic Fatty Liver Disease? Gastroenterology 2020; 158(7): 1929-47 e6.
(28). Liver immunology and its role in inflammation and homeostasis. Cell Mol Immunol 2016; 13(3): 267-76.
(29). Non-alcoholic fatty liver disease and risk of incident chronic kidney disease: an updated meta-analysis. Gut 2022; 71(1): 156-62.
(30). Association of metabolic dysfunction-associated fatty liver disease with kidney disease. Nat Rev Nephrol 2022; 18(4): 259-68.
(31). High peritoneal permeability predisposes to hepatic steatosis in diabetic continuous ambulatory peritoneal dialysis patients receiving intraperitoneal insulin. Perit Dial Int 2000; 20(6): 637-42.
(32). The impact of intraperitoneal glucose and insulin on the liver. Perit Dial Int 1996; 16 Suppl 1: S211-4.
(33). Nonalcoholic Fatty Liver Disease (NAFLD)-A New Cardiovascular Risk Factor in Peritoneal Dialysis Patients. Perit Dial Int 2016; 36(4): 427-32.
(34). Liver enzymes in patients with chronic kidney disease undergoing peritoneal dialysis and hemodialysis. Clinics (Sao Paulo) 2012; 67(2): 131-4.
(35). Peritoneal dialysis in liver disorders. Perit Dial Int 1996; 16 Suppl 1: S215-9.
(36). Preconditioning with L-alanyl-glutamine reduces hepatic ischemia-reperfusion injury in rats. Acta Cir Bras 2011; 26 Suppl 1: 8-13.
(37). Alanyl-Glutamine Protects against Lipopolysaccharide-Induced Liver Injury in Mice via Alleviating Oxidative Stress, Inhibiting Inflammation, and Regulating Autophagy. Antioxidants (Basel) 2022; 11(6).
(38). Alanyl-Glutamine Protects Mice against Methionine- and Choline-Deficient-Diet-Induced Steatohepatitis and Fibrosis by Modulating Oxidative Stress and Inflammation. Nutrients 2022; 14(18).
(39). CKD and nonalcoholic fatty liver disease. Am J Kidney Dis 2014; 64(4): 638-52.
(40). Nonalcoholic fatty liver disease increases risk of incident chronic kidney disease: A systematic review and meta-analysis. Metabolism 2018; 79: 64-76.
(41). Association of non-alcoholic fatty liver disease with chronic kidney disease: a systematic review and meta-analysis. PLoS medicine 2014; 11(7): e1001680.
(42). MAFLD and risk of CKD. Metabolism 2021; 115: 154433.
(43). Higher liver stiffness scores are associated with early kidney dysfunction in patients with histologically proven non-cirrhotic NAFLD. Diabetes Metab 2020; 46(4): 288-95.
(44). Association between the fatty liver index and chronic kidney disease: the population-based KORA study. Nephrol Dial Transplant 2022.
(45). Prevalence of non-alcoholic fatty liver disease among patients with non-diabetic chronic kidney disease detected by transient elastography. Int Urol Nephrol 2021; 53(12): 2593-601.
(46). Associations between liver function parameters and poor clinical outcomes in peritoneal dialysis patients. Ther Apher Dial 2022.
(47). Design and validation of a histological scoring system for nonalcoholic fatty liver disease. Hepatology 2005; 41(6): 1313-21.

## Claims

1. A protective agent selected from the group consisting of L-glutamine, L-alanyl-L-glutamine, L-glutaminyl-L-alanine, L-glutaminyl-L-glycine, L-glycinyl-L-glutamine or mixtures thereof, for the specific use in the prevention and/or treatment, in the course of a peritoneal dialysis treatment, of
(A) liver diseases induced by said peritoneal dialysis treatment and/or
(B) diseases associated with end-stage kidney disease (ESKD) mediated by liver dysfunction induced by said peritoneal dialysis treatment,
whereby said protective agent is administered by intraperitoneal administration.

2. Protective Agent for use according to claim 1, wherein the liver diseases induced by said peritoneal dialysis treatment (A) are selected from the group consisting of chronic liver disease, especially non-infectious liver inflammation.

3. Protective Agent for use according to claim 1, wherein said non-infectious liver inflammation includes non-alcoholic fatty liver disease (NAFLD), non-alcoholic steatohepatitis (NASH), and metabolic (dysfunction)-associated fatty liver disease (MAFLD).

4. Protective Agent for use according to any one of the preceding claims, wherein said ESKD-associated diseases mediated by liver dysfunction (B) are selected from the group consisting of cardiovascular diseases, especially artherosclerotic cardiovascular diseases, including inflammation driven cardiovascular diseases and metaflammation driven cardiovascular diseases.

5. Protective Agent for use according to any one of the preceding claims, wherein administration of the protective agent starts from the outset of said peritoneal dialysis treatment.

6. Protective Agent for use according to any one of claims 1 to 4, wherein administration of the protective agent starts after a period from the outset of said peritoneal dialysis treatment.

7. Protective Agent for use according to claim 6, wherein administration of the protective agent starts once indicators for liver disease or liver-mediated system inflammation, especially metaflammation, or cardiocascular disease, are detected.

8. Protective Agent for use according to claim 7, wherein said indicators are selected from the group consisting of biomarkers, such as laboratory markers, and/or imaging findings.

9. Protective agent for use according to any of the preceding claims, whereby the protective agent is administered as a component of a peritoneal dialysis fluid (PDF).

10. Protective agent for use according to claim 9 wherein the fluid is administered as the only PDF in the course of the PD treatment.

11. Protective agent for use according to claim 9, wherein the fluid is administered in combination with another peritoneal dialysis fluid.

12. Protective agent for use according to any one of claims 9 to 11, wherein the fluid is based on glucose as the osmotic agent.

13. Protective agent for use according to any one of the preceding claims, wherein the protective agent is L-alanyl-L-glutamine, optionally in mixture with one or more of the other protective agents.

## Amended claims

### Amended claims in accordance with Rule 137(2) EPC.

1. L-alanyl-L-glutamine for the specific use in the prevention and/or treatment, in the course of a peritoneal dialysis treatment, of
(A) liver diseases induced by said peritoneal dialysis treatment and/or
(B) cardiovascular diseases mediated by liver dysfunction induced by said peritoneal dialysis treatment,
whereby said protective agent is administered by intraperitoneal administration,
wherein in the case of treatment, administration of the protective agent starts after a period from the outset of said peritoneal dialysis treatment, once indicators for liver disease or liver-mediated system inflammation, especially metaflammation, or cardiocascular disease, are detected.

2. Protective Agent for use according to claim 1, wherein the liver disease induced by said peritoneal dialysis treatment (A) is chronic liver disease, especially non-infectious liver inflammation.

3. Protective Agent for use according to claim 2, wherein said non-infectious liver inflammation includes non-alcoholic fatty liver disease (NAFLD), non-alcoholic steatohepatitis (NASH), and metabolic (dysfunction)-associated fatty liver disease (MAFLD).

4. Protective Agent for use according to any one of the preceding claims, wherein said ESKD-associated disease mediated by liver dysfunction (B) is an artherosclerotic cardiovascular disease, including inflammation driven cardiovascular disease and metaflammation driven cardiovascular disease.

5. Protective Agent for use according to claim 1, wherein said indicators are selected from the group consisting of biomarkers, such as laboratory markers, and/or imaging findings.

6. Protective agent for use according to any of the preceding claims, whereby the protective agent is administered as a component of a peritoneal dialysis fluid (PDF).

7. Protective agent for use according to claim 6 wherein the fluid is administered as the only PDF in the course of the PD treatment.

8. Protective agent for use according to claim 6, wherein the fluid is administered in combination with another peritoneal dialysis fluid.

9. Protective agent for use according to any one of claims 6 to 8, wherein the fluid is based on glucose as the osmotic agent.
